# FASCICULE DE BREVET EUROPEEN

(11) **EP 0 819 405 B1**
(45) Date de publication et mention de la délivrance du brevet: **12.11.2003**
(21) Numéro de dépôt: 97401659.4
(22) Date de dépôt: 10.07.1997
(51) Int. Cl.: A61B 5/0205

(54) **Module pour enregister la fréquence cardiaque instantanée et module portable en faisant application**
Modul zur Wiedergabe der Augenblicksherzfrequenz und die Verwendung als tragbares Modul
Module for recording the instantaneous cardiac frequency and its use as a portable module

(30) Priorité: 18.07.1996 FR 9608993
(43) Date de publication de la demande: 21.01.1998
(73) Titulaire: ETAT FRANCAIS représenté par le Délégué Général pour l'Armement, F-00460 Armées (FR)
(72) Inventeur: Savourey, Gustave, 38330 Biviers (FR); Caterini, Richard, 69530 Orlienas (FR)

(56) Documents cités:
- EP-A- 0 553 372
- DE-A- 2 633 371
- US-A- 4 576 178

## Description

La présente invention concerne le domaine technique de l'enregistrement de données physiologiques et physiques d'un patient en vue de permettre de procéder, en particulier, à des surveillances, recherches ou diagnostics.

L'objet de l'invention vise plus précisément à enregistrer, d'une part, l'activité électrique du muscle cardiaque d'un patient et, d'autre part, la température du patient.

Dans le domaine technique ci-dessus, il est connu de nombreux appareils permettant, d'une part, de recueillir la température du patient et, d'autre part, de délivrer, par l'intermédiaire de cellules placées sur le corps d'un patient, un signal d'électrocardiogramme, dit ECG, correspondant aux courants électriques produits par les contractions du muscle cardiaque. Il a été mis au point une gamme d'appareils présentant l'inconvénient d'être fixes et de courte autonomie, ce qui impose une acquisition des données à des moments ne correspondant pas forcément à une phase caractéristique du rythme cardiaque. De plus, l'enregistrement des données est réalisé alors que la personne se trouve dans des conditions qui ne reproduisent pas exactement les situations réelles vécues au quotidien par le patient. Il est ainsi apparu le besoin de disposer d'un dispositif ambulatoire destiné à être adapté sur un patient et conçu pour permettre d'acquérir pendant une période de plusieurs jours à plusieurs semaines, aussi bien des données physiques que physiologiques.

Le document européen EP-A-0 553 372 divulge un tel dispositif ambulatoire permettant d' acquérir au moins deux signaux de mesure tel un signal ECG et un signal de température.

Un des inconvénients des dispositifs à caractère ambulatoire concerne leur caractère d'imprécision, dans la mesure où ils ne peuvent pas suivre la fréquence cardiaque instantanée. De plus, ces dispositifs présentent généralement une faible autonomie limitant leur temps d'utilisation.

L'objet de l'invention vise à remédier aux inconvénients énoncés ci-dessus en proposant un procédé pour enregistrer la fréquence cardiaque instantanée d'un patient ainsi que sa température.

Pour atteindre cet objectif, le procédé selon l'invention consiste :
- détecter les pics du signal ECG à l'aide de deux intégrateurs présentant des constantes de temps différentes et raccordées à un comparateur délivrant un signal représentatif des cycles cardiaques,
- enregistrer la durée des cycles cardiaques,
- et à enregistrer la température dès la détection du début des cycles cardiaques.

Un autre objet de l'invention vise à proposer un module portable permettant l'enregistrement de la fréquence cardiaque instantanée et la température du patient tout en présentant une grande autonomie de fonctionnement.

Selon l'invention, le module comporte :
- une source principale d'énergie reliée à une source secondaire d'énergie alimentant par un circuit commandé, le capteur de température,
- une unité d'acquisition et de traitement du signal ECG comportant un étage de filtration du signal ECG, relié à deux intégrateurs présentant des constantes de temps différentes, les sorties des intégrateurs étant connectées à un comparateur délivrant un signal représentatif des cycles cardiaques,
- des moyens de mise en fonctionnement de l'unité d'acquisition et de traitement du signal ECG,
- et un processeur comportant :
   . un convertisseur analogique-numérique destiné à recevoir le signal analogique délivré par le capteur de température,
   . un compteur auquel est reliée la sortie du comparateur, afin de permettre de déterminer la durée des cycles cardiaques,
   . des moyens de commande permettant, d'une part, de commander le circuit pilotant le fonctionnement de la source secondaire d'énergie et, d'autre part, d'enregistrer la durée des cycles cardiaques et les valeurs de température associées.

Diverses autres caractéristiques ressortent de la description faite ci-dessous en référence aux dessins annexés qui montrent, à titre d'exemples non limitatifs, des formes de réalisation et de mise en oeuvre de l'objet de l'invention.

La **fig. 1** est un schéma-bloc fonctionnel illustrant le module ambulatoire selon l'invention.

La **fig. 2** est un schéma illustrant un détail caractéristique du module ambulatoire conforme à l'invention.

La **fig. 3** est un exemple de courbes illustrant le principe de détection du rythme cardiaque, conforme à l'invention.

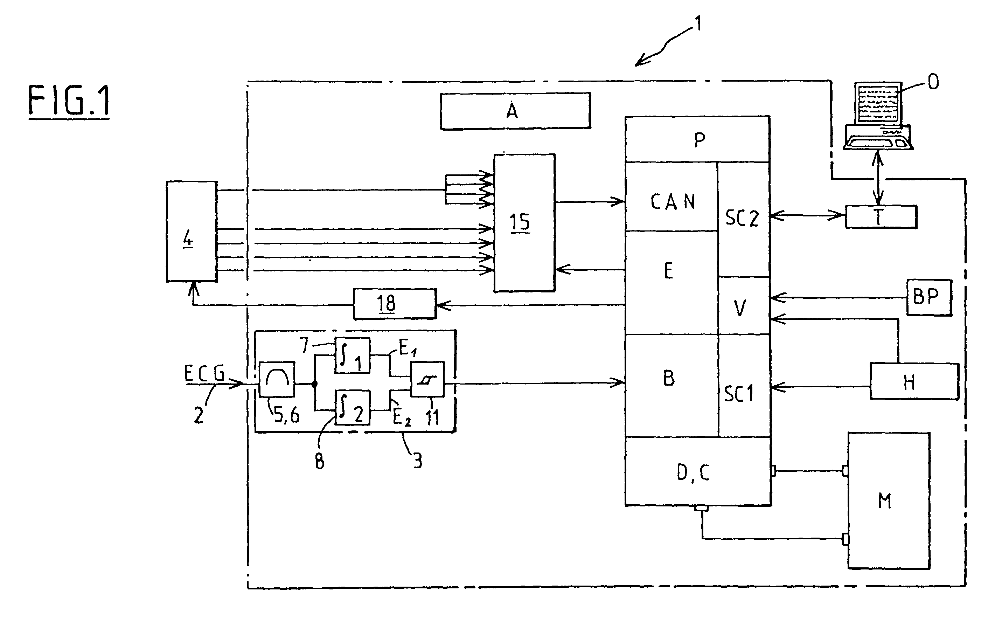

Tel que cela ressort plus précisément de la **Fig. 1**, le module **1** est adapté pour enregistrer l'activité cardiaque et la température d'une personne. Le module **1** est destiné à être porté par le patient par tous moyens appropriés non représentés. Le module portable **1** est conçu pour être relié à un dispositif non représenté mais connu en soi comportant des électrodes placées sur le patient, de manière à délivrer un signal électrique produit par les contractions du muscle cardiaque. Un tel signal d'électrocardiogramme **2**, dit ECG dans la suite de la description, est reçu par une unité d'acquisition et de traitement **3** faisant partie du module **1**. Conformément à l'invention, le module **1** est destiné à recueillir également au moins une température du patient simultanément à l'acquisition du signal ECG. Le module **1** est ainsi destiné à être connecté à des capteurs **4** placés de manière à permettre de recueillir par exemple une ou plusieurs températures cutanées, oesophagiennes, tympaniques ou rectales.

Le module 1 comporte des moyens de traitement et de calcul des signaux reçus et se trouve ainsi architecturé autour d'un processeur **P**, par exemple du type Microchip Technology Inc. PIC 16C74. Le module **1** comporte également une alimentation principale **A** délivrant, par exemple, une tension de 3,6 V, un bloc mémoire **M** relié au port de sortie **D, C** du processeur **P**, une horloge **H** connectée à un port série **SC1** du processeur et un circuit de transmission **T** relié au port série **SC2** du processeur et destiné à être relié avec un ordinateur délocalisé **O** par l'intermédiaire d'une liaison, par exemple, RS232. Le module **1** est également pourvu d'un bouton poussoir **BP** connecté à l'unité de veille **V** du processeur **P**.

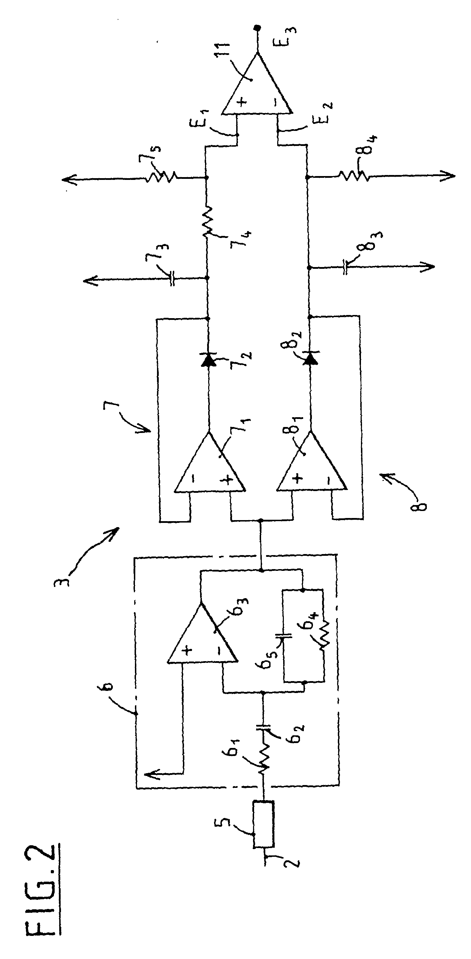

Le processeur **P** comporte également un compteur **B** relié à l'unité d'acquisition et de traitement **3**. Tel que cela ressort plus précisément de la **fig. 2**, l'unité d'acquisition et de traitement **3** comporte, en entrée, un étage **5** de filtrage du signal ECG. L'étage de filtrage **5** présente une bande passante comprise entre 1 et 24 Hz et de préférence, entre 5 et 16 Hz. La sortie de l'étage de filtrage **5** est reliée à un circuit **6** d'amplification de la composante alternative du signal. Ainsi, la sortie de l'étage de filtrage **5** est reliée par l'intermédiaire d'une résistance **6**_{**1**} et d'un condensateur **6**_{**2**} montés en série, à l'entrée inverseuse d'un amplificateur opérationnel **6**_{**3**} dont l'entrée non inverseuse est reliée à la masse. L'entrée inverseuse de l'amplificateur opérationnel **6**_{**3**} est reliée à sa sortie par l'intermédiaire d'un circuit parallèle résistif **6**_{**4**} et capacitif **6**_{**5**}. La sortie de l'amplificateur opérationnel **6**_{**3**} et, par suite, de l'étage **6** est connectée à deux intégrateurs **7** et **8** présentant des constantes de temps différentes.

Chaque intégrateur **7** et **8** comporte ainsi un amplificateur opérationnel **7**_{**1**}, **8**_{**1**} dont l'entrée non inverseuse est reliée à la sortie de l'étage de filtrage **6**. L'entrée inverseuse de chaque amplificateur **7**_{**1**}**, 8**_{**1**} est connectée à la cathode d'une diode **7**_{**2**}**, 8**_{**2**} dont l'anode est reliée à la sortie de l'amplificateur opérationnel correspondant. La cathode de chaque diode **7**_{**2**}**, 8**_{**2**} est reliée à la masse par l'intermédiaire d'une capacité **7**_{**3**}**, 8**_{**3**} montée en parallèle respectivement avec des résistances **7**_{**4**}**, 7**_{**5**} et **8**_{**4**}. Chaque intégrateur **7**, **8** délivre ainsi un signal **E**_{**1**}**, E**_{**2**} présentant respectivement des constantes de temps τ₁, τ₂ de valeurs différentes. Par exemple, le signal **E**_{**1**} présente une constante de temps égale à τ₁ = **3** secondes, tandis que le signal **E**_{**2**} présente une constante de temps τ₂ = **0,2** seconde. Les sorties des intégrateurs **7**, **8** attaquent un comparateur **11** délivrant en sortie un signal **E**_{**3**} en créneaux représentatif du cycle cardiaque.

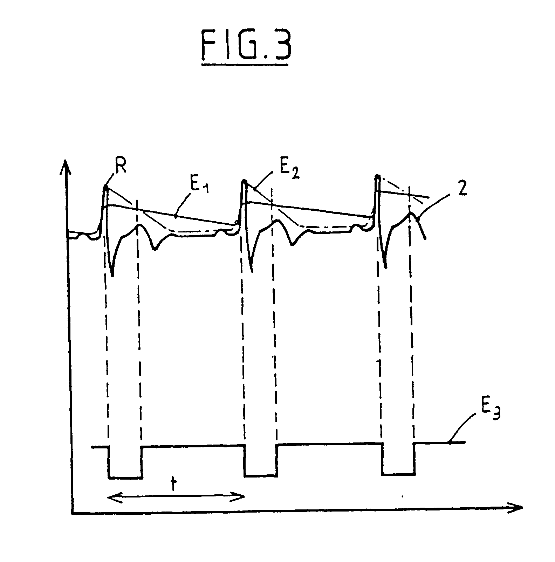

Tel que cela ressort plus précisément de la **fig. 3**, l'unité d'acquisition et de traitement **3** permet de détecter les pics "R" du signal ECG à l'aide des deux signaux **E**_{**1**} et **E**_{**2**} détectant l'enveloppe du signal avec des fidélités différentes. Ainsi, le comparateur **11** passe d'un niveau haut à un niveau bas, dès que le signal **E**_{**1**} qui possède la constante de temps la plus grande, a atteint sa valeur maximale. Le comparateur **11** conserve son état bas tant que la valeur du signal **E**_{**2**} est supérieure à la valeur du signal **E**_{**1**}. Dès que la valeur du signal **E**_{**2**} devient inférieure à la valeur du signal **E**_{**1**}, le comparateur **11** bascule d'un niveau bas à un niveau haut. L'unité d'acquisition et de traitement **3** permet ainsi de détecter avec une grande fidélité les pics "R" du signal ECG et, par suite, de détecter le rythme cardiaque d'une personne.

Ainsi, la mesure de chaque période élémentaire **t** séparant deux pulsations cardiaques consécutives, combinée au comptage du nombre de pics ou de pulsations, permet de déterminer la fréquence cardiaque instantanée. La fréquence cardiaque instantanée est obtenue en effectuant la somme des périodes élémentaires **t**, divisée par le nombre de pics cardiaques déterminés. Il est à noter qu'il peut être envisagé de déterminer cette fréquence cardiaque instantanée en considérant non plus la période élémentaire **t**, mais une période **T** correspondant à n x **t**.

Tel que cela ressort plus précisément de la **fig. 1**, le processeur **P** comporte un convertisseur analogique-numérique **CAN** connecté à un multiplexeur **15** dont les entrées analogiques sont reliées aux capteurs de température **4**. Le multiplexeur **15** est piloté par l'intermédiaire d'un signal délivré, par exemple, par le port de sortie **E** du processeur **P**. Selon une caractéristique de l'invention, le port **E** commande une source d'énergie secondaire **18** destinée à alimenter les capteurs de température **4** de tous types connus en soi.

Le module portable **1** décrit ci-dessus est mis en oeuvre de la manière suivante en considérant que le processeur **P** intègre des moyens de programmation adaptés pour assurer son fonctionnement conformément à l'invention.

Lors de son non fonctionnement, il doit être considéré que le processeur **P** est placé en état de sommeil, tandis que la source d'énergie secondaire **18** n'alimente pas les capteurs de température **4**. Le processeur **P** comporte des moyens de programmation intégrés permettant de surveiller les interruptions susceptibles d'intervenir par l'horloge **H**, le bouton poussoir **BP** ou le système informatique **O**. Préalablement à la réalisation d'une série de mesures, le module **1** reçoit des consignes ou des paramètres de fonctionnement par l'intermédiaire du système informatique **O**. Cette phase d'initialisation du module **1** consiste, notamment, à définir les consignes d'enregistrement, à savoir l'heure de début et de fin de l'enregistrement des mesures, ainsi que la définition du nombre n de périodes cardiaques à prendre en compte. Concernant ce dernier paramètre, il peut être prévu de sélectionner la valeur n entre 1 et 16, avec **T** = n × **t** et **t**, la période élémentaire entre deux pulsations consécutives. Le module offre ainsi l'avantage de choisir la fréquence de sélection des cycles cardiaques entre les valeurs 1 et 16. Plus la valeur de n est élevée, plus il apparaît possible d'augmenter la durée de l'enregistrement pour une même capacité de stockage des mesures. Cependant, selon cette hypothèse, moins la mesure effectuée est fine, car chaque période cardiaque mesurée correspond à une moyenne pour n pulsations cardiaques. En d'autres termes, il peut être considéré que le module permet de mesurer la fréquence cardiaque instantanée correspondant à la fréquence cardiaque instantanée réelle ou affectée d'un facteur de pondération n.

Après enregistrement des consignes, le module **1** est adapté pour scruter l'état du bouton poussoir **BP** et lire l'horloge, afin de déterminer si l'heure de début d'enregistrement des données choisies lors de la phase d'initialisation est atteinte. Dès que l'heure de début de la période d'enregistrement est atteinte ou qu'une action sur le bouton poussoir intervient, le processeur **P** passe en mode sommeil et se trouve placé en état d'attente d'une interruption générée par le compteur et correspondant à un niveau bas du signal ECG. Dès la détection du niveau bas du signal ECG, le processeur **P** lit une horloge **H**_{**1**} et commande la source d'énergie secondaire **18** alimentant les capteurs de température **4**. Le multiplexeur **15** est commandé de manière à permettre l'enregistrement des températures sélectionnées. Dès la fin de l'acquisition des températures, la source d'énergie secondaire **18** est commandée de manière à ne plus alimenter les capteurs **4**. Le processeur **P** passe alors en mode sommeil jusqu'à une nouvelle interruption. Dès l'apparition d'un nouveau niveau bas du signal **E**_{**3**} correspondant à la n^{ième} pulsation consécutive à celle précédemment détectée, les valeurs des capteurs de température sont acquises et une horloge **H**_{**2**} est lue. Il est procédé à la différence des valeurs **H**_{**2**} et **H**_{**1**} pour déterminer la durée T d'un cycle sélectionné. Comme expliqué précédemment, la fréquence cardiaque instantanée est obtenue en divisant la durée **T** par le paramètre n sélectionné.

Le module ambulatoire selon l'invention permet de détecter avec une grande précision la fréquence cardiaque instantanée tout en offrant une grande autonomie de fonctionnement. Ainsi, pour n = 1, il peut être possible d'effectuer des mesures sur une période de deux mois, tandis qu'une sélection d'une valeur n supérieure permet de multiplier d'autant la période de mesure.

L'invention n'est pas limitée aux exemples décrits et représentés, car diverses modifications peuvent y être apportées sans sortir de son cadre.

## Revendications

1. Procédé pour enregistrer, d'une part, l'activité cardiaque d'une personne à l'aide d'un dispositif délivrant un signal ECG et, d'autre part, au moins une température du patient à l'aide d'un capteur de température (**4**), **caractérisé en ce qu'**il consiste
- à détecter les pics "R" du signal ECG à l'aide de deux intégrateurs (**7, 8**) présentant des constantes de temps différentes et raccordées à un comparateur (**11**) délivrant un signal (**E**_{**3**}) représentatif des cycles cardiaques,
- à enregistrer la durée (**T**) des cycles cardiaques, en vue de déterminer, en considération du nombre de pics détectés, la fréquence cardiaque instantanée,
- et à enregistrer la température dès la détection du début des cycles cardiaques.

2. Procédé selon la revendication 1, **caractérisé en ce qu'**il consiste à choisir une fréquence (n) de sélection des cycles cardiaques et à enregistrer la durée des cycles cardiaques sélectionnés.

3. Procédé selon la revendication 1, **caractérisé en ce qu'**il consiste à alimenter en énergie les capteurs de température (4) dès la détection du début de chaque cycle cardiaque et à l'interrompre dès l'enregistrement des valeurs de températures.

4. Procédé selon la revendication 1, **caractérisé en ce qu'**il consiste à assurer le filtrage du signal ECG, dont la bande passante est comprise entre 1 et 24 Hz et, de préférence, entre 5 et 16 Hz.

5. Module portable pour enregistrer, selon le procédé conforme à la revendication 1, d'une part, l'activité cardiaque d'une personne à l'aide d'un dispositif délivrant un signal ECG et, d'autre part, au moins une température du patient prise à l'aide d'un capteur de température (**4**), **caractérisé en ce qu'**il comporte :
- une source principale d'énergie (**A**) reliée à une source secondaire d'énergie (**18**) alimentant par un circuit commandé, le capteur de température (4),
- une unité (**3**) d'acquisition et de traitement du signal ECG comportant un étage de filtrage (**5**) du signal ECG, relié à deux intégrateurs (**7, 8**) présentant des constantes de temps différentes, les sorties des intégrateurs étant connectées à un comparateur (**11**) délivrant un signal (**E**_{**3**}) représentatif des cycles cardiaques,
- des moyens de mise en fonctionnement de l'unité d'acquisition et de traitement du signal ECG,
- et un processeur (**P**) comportant :
. un convertisseur analogique-numérique (CAN) destiné à recevoir le signal analogique délivré par le capteur de température,
. un compteur (**B**) auquel est reliée la sortie du comparateur (**11**), afin de permettre de déterminer la durée des cycles cardiaques,
. des moyens de commande permettant, d'une part, de commander le circuit pilotant le fonctionnement de la source secondaire d'énergie (**18**) et, d'autre part, d'enregistrer la durée des cycles cardiaques et les valeurs de température associées,
. et des moyens de détermination de la fréquence cardiaque instantanée en considération du nombre de pics détectés et de la durée des cycles cardiaques.

6. Module selon la revendication 5, **caractérisé en ce qu'**il comporte des moyens de sélection des consignes d'enregistrement liées aux cycles cardiaques permettant de choisir une fréquence (n) de sélection des cycles cardiaques et d'enregistrer la durée des cycles cardiaques sélectionnés.

7. Module selon la revendication 6, **caractérisé en ce que** les moyens de sélection des consignes d'enregistrement permettent de choisir l'enregistrement des durées des cycles cardiaques dans une gamme de fréquence de sélection comprise entre 1 à 16.

8. Module selon la revendication 5, **caractérisé en ce que** les moyens de mise en fonctionnement de l'unité d'acquisition et de traitement du signal ECG sont constitués par un bouton de commande (**BP**) ou une horloge programmée (**H**).

9. Module selon la revendication 5, **caractérisé en ce que** l'unité d'acquisition et de traitement comporte un circuit de filtrage du signal ECG dont la bande passante est comprise entre 1 et 24 Hz et, de préférence, entre 5 et 16 Hz.

## Claims

1. A process for recording, on the one hand, the cardiac activity of a person with the aid of a device delivering an ECG signal and, on the other hand, at least one temperature of the patient with the aid of a temperature sensor (4), **characterised in that** it consists in:
- detecting the peaks "R" of the ECG signal with the aid of two integrators (7, 8) which have different time constants and which are connected up to a comparator (11) delivering a signal (E₃) that is representative of the cardiac cycles,
- recording the duration (T) of the cardiac cycles with a view to determining the instantaneous heart rate by considering the number of peaks detected,
- and recording the temperature as soon as the start of the cardiac cycles is detected.

2. Process according to Claim 1, **characterised in that** it consists in choosing a selection frequency (*n*) of the cardiac cycles and in recording the duration of the selected cardiac cycles.

3. Process according to Claim 1, **characterised in that** it consists in feeding the temperature sensors (4) with energy as soon as the start of each cardiac cycle is detected and in interrupting this feeding as from the recording of the temperature values.

4. Process according to Claim 1, **characterised in that** it consists in ensuring the filtration of the ECG signal, the pass-band of which lies between 1 and 24 Hz, preferably between 5 and 16 Hz.

5. A portable module for recording, in accordance with the process according to Claim 1, on the one hand, the cardiac activity of a person with the aid of a device delivering an ECG signal and, on the other hand, at least one temperature of the patient taken with the aid of a temperature sensor (4), **characterised in that** it comprises:
- a principal source of energy (A) linked to a secondary source of energy (18) feeding the temperature sensor (4) by means of a controlled circuit,
- a unit (3) for acquisition and processing of the ECG signal including a stage for filtration (5) of the ECG signal, linked to two integrators (7, 8) having different time constants, the outputs of the integrators being connected to a comparator (11) delivering a signal (E₃) that is representative of the cardiac cycles,
- means for putting the unit for acquisition and processing of the ECG signal into operation,
- and a processor (P) including:
- an analogue/digital converter (CAN) intended to receive the analogue signal delivered by the temperature sensor,
- a counter (B), to which the output of the comparator (11) is linked in order to permit the duration of the cardiac cycles to be determined,
- control means permitting, on the one hand, the circuit driving the operation of the secondary source of energy (18) to be controlled, and, on the other hand, the duration of the cardiac cycles and the associated temperature values to be recorded,
- and means for determining the instantaneous heart rate by considering the number of peaks detected and the duration of the cardiac cycles.

6. Module according to Claim 5, **characterised in that** it includes means for selection of the set points of recording which are linked to the cardiac cycles permitting a selection frequency (*n*) of the cardiac cycles to be chosen and the duration of the selected cardiac cycles to be recorded.

7. Module according to Claim 6, **characterised in that** the means for selection of the set points of recording permit the recording of the durations of the cardiac cycles to be chosen within a selection-frequency range between 1 and 16.

8. Process according to Claim 5, **characterised in that** the means for putting the unit for acquisition and processing of the ECG signal into operation are constituted by a control button (BP) or a programmed clock (H).

9. Process according to Claim 5, **characterised in that** the unit for acquisition and processing includes a circuit for filtration of the ECG signal, the pass-band of which lies between 1 and 24 Hz, preferably between 5 and 16 Hz.

## Patentansprüche

1. Verfahren zum Aufzeichnen einerseits der Herzaktivität einer Person mit Hilfe einer Vorrichtung, die ein EKG-Signal liefert, und andererseits wenigstens einer Temperatur des Patienten mit Hilfe eines Temperatursensors (4), **dadurch gekennzeichnet, daß** es folgende Schritte umfaßt:
- Ausfindigmachen der Spitzen "R" des EKG-Signals mit Hilfe von zwei Integratoren (7, 8), die verschiedene Zeitkonstanten aufweisen und mit einem Komparator (11) verbunden sind, der ein für die Herzzyklen repräsentatives Signal (E₃) liefert,
- Aufzeichnen der Dauer (T) der Herzzyklen, um unter Berücksichtigung der Anzahl der festgestellten Spitzen die augenblickliche Herzfrequenz zu bestimmen,
- und Aufzeichnen der Temperatur, sobald der Beginn eines Herzzyklus detektiert wurde.

2. Verfahren nach Anspruch 1, **dadurch gekennzeichnet, daß** es die Wahl einer Frequenz (n) für die Auswahl von Herzzyklen und die Aufzeichnung der Dauer der ausgewählten Herzzyklen umfaßt.

3. Verfahren nach Anspruch 1, **dadurch gekennzeichnet, daß** die Temperatursensoren (4) mit Energie versorgt werden, sobald von jedem Herzzyklus der Beginn festgestellt wird, und die Energiezufuhr unterbrochen wird, sobald die Temperaturwerte aufgezeichnet sind.

4. Verfahren nach Anspruch 1, **dadurch gekennzeichnet, daß** es die Filterung des EKG-Signals vornimmt, dessen Bandbreite zwischen 1 und 24 Hz und vorzugsweise zwischen 5 und 16 Hz liegt.

5. Tragbares Modul um nach dem Verfahren entsprechend des Anspruchs 1 einerseits die Herzaktivität einer Person mit Hilfe einer Vorrichtung, die ein EKG-Signal liefert, und andererseits mindestens eine Temperatur des Patienten, die mit Hilfe eines Temperatursensors (4) genommen wird, aufzuzeichnen, **dadurch gekennzeichnet, daß** es folgendes umfaßt:
- eine Hauptenergiequelle (A), die mit einer Zweitenergiequelle (18) verbunden ist, die über einen gesteuerten Schaltkreis den Temperatursensor (4) versorgt,
- eine Einheit (3) zur Erfassung und Verarbeitung des EKG-Signals, die eine Filterstufe (5) zum Filtern des EKG-Signals umfaßt, die mit zwei Integratoren (7, 8) mit unterschiedlichen Zeitkonstanten verbunden ist, wobei die Ausgänge der Integratoren mit einem Komparator (11) verbunden sind, der ein für die Herzzyklen repräsentatives Signal (E₃) liefert,
- Mittel zum Inbetriebsetzen der Einheit zur Erfassung und Verarbeitung des EKG-Signals,
- und einen Prozessor (P), der folgendes umfaßt:
einen Analog-Digital-Converter (CAN), um das vom Temperatursensor gelieferte analoge Signal zu empfangen,
einen Zähler (B), mit dem der Ausgang des Komparators (11) verbunden ist, um die Bestimmung der Dauer der Herzzyklen zu erlauben,
Mittel zur Steuerung, die es einerseits erlauben, den Schaltkreis zu steuern, der den Betrieb der Zweitenergiequelle (18) regelt, und es andererseits erlauben, die Dauer der Herzzyklen und die dazugehörigen Temperaturwerte aufzuzeichnen,
und Mittel zur Bestimmung der augenblicklichen Herzfrequenz unter Berücksichtigung der Anzahl der festgestellten Spitzen und der Dauer der Herzzyklen.

6. Modul nach Anspruch 5, **dadurch gekennzeichnet, daß** es Auswahlmittel für die Aufzeichnungsvorgaben im Zusammenhang mit den Herzzyklen umfaßt, die es erlauben, eine Frequenz (n) zur Auswahl von Herzzyklen zu wählen und die Dauer der ausgewählten Herzzyklen aufzuzeichnen.

7. Modul nach Anspruch 6, **dadurch gekennzeichnet, daß** die Auswahlmittel für die Aufzeichnungsvorgaben es erlauben, die Aufzeichnung der Dauern von Herzzyklen in einem Auswahlfrequenzbereich zu wählen, der zwischen 1 und 16 liegt.

8. Modul nach Anspruch 5, **dadurch gekennzeichnet, daß** die Mittel zur Inbetriebnahme der Einheit zur Erfassung und Verarbeitung des EKG-Signals aus einer Befehlstaste (BP) oder einer programmierten Uhr (H) gebildet werden.

9. Modul nach Anspruch 5, **dadurch gekennzeichnet, daß** die Einheit zur Erfassung und Verarbeitung einen Schaltkreis zur Filterung des EKG-Signals umfaßt, dessen Bandbreite zwischen 1 und 24 Hz und vorzugsweise zwischen 5 und 16 Hz liegt.
